# FASCICULE DE BREVET EUROPEEN

(11) **EP 0 796 238 B1**
(45) Date de publication et mention de la délivrance du brevet: **17.05.2000**
(21) Numéro de dépôt: 95925889.8
(22) Date de dépôt: 17.07.1995
(51) Int. Cl.: C07C 57/12, A61K 31/20, C07C 51/50

(54) **PROCEDE DE STABILISATION DES ACIDES GRAS POLY-INSATURES ET UTILISATION DE CES PRODUITS STABILISES EN THERAPEUTIQUE ET EN COSMETOLOGIE**
VERFAHREN ZUR STABILISATION DER POLYUNGESÄTTIGTEN FETTSÄUREN UND VERWENDUNG DIESER STABILISIERTEN PRODUKTEN IN THERAPIE UND IN KOSMETOLOGIE
METHOD OF STABILIZING POLYUNSATURATED FATTY ACIDS AND THEIR APPLICATION IN THERAPY AND COSMETICS

(43) Date de publication de la demande: 24.09.1997
(73) Titulaire: Grinda, Jean-Robert, F-75016 Paris (FR)
(72) Inventeur: Grinda, Jean-Robert, F-75016 Paris (FR)
(74) Mandataire: Burtin, Jean-François
(86) Numéro de dépôt international: FR9500949
(87) Numéro de publication internationale: WO9602488

(56) Documents cités:
- FR-A- 2 573 653
- US-A- 2 827 452
- PATENT ABSTRACTS OF JAPAN vol. 10, no. 131 (C-346) (2188) 15 Mai 1986 & JP,A,60 258 139 (NIPPON YUSHI K.K.) 20 Décembre 1985

## Description

La présente invention se rapporte au domaine de la pharmacie, plus particulièrement à celui de la chimie thérapeutique et à celui de la dermato-cosmétologie.

Elle a plus particulièrement pour objet, un procédé de stabilisation des acides gras poly-insaturés en vue d'une utilisation thérapeutique sûre et prolongée.

L'intérêt pour les acides poly-insaturés, notamment l'acide α ou l'acide γ-linolénique (vitamine F), a été en croissant depuis quelques années en raison de leur position particulière dans les diverses synthèses biologiques. C'est ainsi que les acides gras poly-insaturés de la série 18:3 et surtout de la série 20:3 ont pu être considérés comme des précurseurs et des intermédiaires clés pour la formation de prostaglandines PGE 1 à la suite d' une série de réactions enzymatiques dues à des désaturases spécifiques et à des élongases.

L'intérêt pour les acides gras non saturés en dermatologie et en cosmétologie a été également en croissant en raison de leurs propriétés régénérantes et anti-vieillissement pour l'épiderme. A cet égard, les propriétés des acides gras polyinsaturés ne dépendent pas d'une transformation éventuelle en prostaglandines ou en prostacyclines, mais d'un effet direct.

L'utilisation en thérapeutique des acides gras polyinsaturés découle de leur possibilité de conversion en prostaglandines, et tout particulièrement PGE₁, qui jouent un rôle de médiateur important dans la régulation de la pression artérielle, dans la motricité et la contractibilité musculaire des muscles lisses et dans la protection contre les ulcères gastriques.

Les espoirs d'utilisation de ces acides ne se sont pas réalisés, d'une part en raison de leur faible degré d'assimilation mais, d'autre part et surtout, par suite de la facile et rapide oxydation des acides gras polyinsaturés sous l'influence de la lumière, des peroxydes et, en particulier, l'oxygène atmosphérique, conduisant à la formation de résines.

En conséquence, les acides gras poly-insaturés comme les acides linoléniques, l'acide stéaridonique, l'acide eicosa pentaenoique ou l'acide dihomo, γ-linolénique ou ses analogues synthétiques, s'oxydent, s'hydroxylent ou se péroxydent facilement, conduisant à des métabolites peu ou pas actifs que l'organisme rejette ou excrète. C'est particulièrement le cas pour l'acide dihomo γ-linolénique (acide cis 8, cis 11, cis 14-eicosatriénoïque), ses analogues synthétiques et pour l'acide arachidonique (acide cis 5, cis 8, cis 11, cis 14-eicosatétraénoique).

Il est donc apparu nécessaire, en vue de l'utilisation therapeutique, de disposer d'un moyen qui empêche l'oxydation de ces acides gras poly-insaturés ou de leurs esters, si l'on désire les maintenir intacts chimiquement et surtout si l'on tient à conserver la possibilité de les voir se métaboliser dans l'organisme en prostaglandines et/ou en prostacyclines. En effet, les produits de décomposition hydroxylés ou oxydés, sont inactifs parce qu'ils ne sont pas résorbés dans le tube digestif ou parce qu'ils ne peuvent plus être transformés par les systèmes enzymatiques en acides gras homologues supérieurs.

Il importait donc de trouver un mode de stabilisation des acides gras poly-insaturés ou de leurs esters, qui leur permette de se conserver sans auto-oxydation et de pouvoir, ainsi, jouer un rôle de pro-drogue des prostaglandines. On sait que l'acide γ-linolénique ou l'acide α-linolénique se transforment rapidement sous l'influence de désaturases spécifiques et d'élongases en acide dihomo γ-linolénique, en acide stéaridonique puis en prostaglandines PGE dont les effets bénéfiques sur la cytoprotection gastrique est reconnue. C'est cette transformation enzymatique qu'il est important de conserver intégralement.

Par la voie topique, les acides gras poly-insaturés jouent également un rôle important concernant le maintien ou la restauration de la jeunesse des téguments ; de nombreux brevets ont montré l'intérêt de préparations cosmétologiques, dermatologiques et nutritionnelles des acides gras poly-insaturés.

L'art antérieur (brevet U.S. 2.827.452) enseigne qu'il est possible de stabiliser des produits chimiques organiques susceptibles de s'auto-oxyder en les incluant dans un complexe d'hydrate de carbone. Parmi ces hydrates de carbone, on a mentionné expressément les alpha - et betadextrines -qui sont en fait les composés dénommés à l'heure actuelle cyclodextrines ou dextrines de Schardinger, les amyloses et les amidons. Ces composés présentent une structure cyclique ou, lorsqu'ils ne sont pas cycliques, possèdent un ajustement de structure à la taille nécessaire en raison de la flexibilité ou de la libre rotation de leur macromolécule.

La formation de tels composés d'inclusion avec des composés auto-oxydables s'effectue en deux étapes : formation d'un précomplexe par chauffage modéré, puis précipitation du complexe d'inclusion par refroidissement. Les complexes formés renferment au maximum de 8 à 10 % du composé hôte en raison du rapport des poids moléculaires. Le composé en excès doit être ensuite éliminé par distillation sous vide poussé.

Le procédé selon l'invention se distingue du procédé décrit antérieurement en ce qu'il n'utilise pas de milieu aqueux pour réaliser la mise en solution du matériau adsorbant, qu'il n'y a pas d'excès de molécule hôte qu'il faut éliminer en fin de réaction et que les hydroxyalkylcelluloses utilisées comme matériau adsorbant, qui ne forment pas de composés d'inclusion, peuvent retenir des quantités d'acide gras polyinsaturé pouvant s'élever jusqu'à 50 % en poids par interposition sur et autour des molécules d'acide gras. Il en résulte une préparation pulvérulente diluable et compressible dont la stabilité pendant plusieurs années a été prouvée.

Notamment on relève de nombreux brevets qui sont intéressants pour montrer l'usage topique des acides gras poly-insaturés, en particulier :
- le brevet (Indena) datant du 27.07.87, IT 2.145.387 (= EP 304.603) intitulé «Polyunsaturated acids having vasokinetic action and pharmaceutical and cosmetic formulations containing them». Ce brevet indique que l'utilisation par voie topique des acides gras essentiels cause, d'une manière surprenante, des effets bénéfiques vasodilateurs et vasokinétiques au niveau veineux, artériolaire et capillaire, et entraîne par là même, un meilleur trophisme cutané.

Par ailleurs, on sait que des acides gras essentiels (en fait des huiles contenant du Gamma linolénate de méthyle) ont été utilisés avec succès dans le traitement de l'eczéma atopique du nourrison, voir par exemple :
- une publication tirée du British Journal of Dermatology ayant pour titre «Changes in transepidermal water loss and the composition of epidermal lecithin after applications of pure fatty acid triglycerides to the skin of essential fatty acid-deficient rats ». La perte d'eau transepidermique caractéristique d'une déficience en acides gras essentiels chez le rat, est réduite par une préparation topique d'acides gras poly-insaturés.

Parmi les (hydroxyalcoyl) celluloses ainsi utilisées, on pourra citer l'hydroxyéthyl et l'hydroxypropyl cellulose. Ce terme inclut également des (hydroxyalcoyl) alcoyl celluloses comme notamment l'hydroxypropylméthyl cellulose.

Les essais effectués ont confirmé que la dispersion d'acide gras poly-insaturé dans une hydroxyalcoyl cellulose et notamment dans l'hydroxypropylméthyl cellulose présentait une stabilité de longue durée pouvant aller au moins jusqu'à quatre ans et que,de ce fait, un des obstacles essentiels à l'utilisation de ces acides gras poly-insaturés se trouvait ainsi éliminé. Ceci paraît inattendu dans la mesure où des dérivés alcoylés de cellulose comme la methylcellulose et l'ethylcellulose ne conduisent qu'à des protections partielles.

L'invention trouve une utilisation particulière et avantageuse dans la stabilisation des analogues semi-synthétiques ou synthétiques de l'acide dihomo γ-linolénique (acide cis 8, cis 11, cis 14-eicosatriénoique) comme ceux décrits dans les publications D.A Van Dorp, Rec. Trav. Chim. 94 (1975) 247-276 et L. HELINGA et al, J. of the Royal Netherlands Chemical Society 94 (1975) 26-29, et en particulier l'acide 19-méthyl eicosatriénoique.

Ces analogues substitués en position 2, 3, 4, 5, 18 ou 19 étaient difficiles à conserver et, en conséquence, leur étude pharmacologique n'avait pas encore pu être réalisée complètement en raison de leur facile aptitude à l'oxydation.

Il semble bien que l'interposition d'une hydroxyalcoyl cellulose entre les particules d'acide gras poly-insaturé ou de leurs esters, amène une disparition rapide et pratiquement complète de l'aptitude à l'oxydation.

L'invention a également pour objet, la réalisation de nouvelles compositions pharmaceutiques contenant un acide gras poly-insaturé, incorporé à un polymère glycosidique, en particulier en association ou en mélange avec un excipient ou un véhicule inerte adapté pour l'administration orale ou topique.

Elle a en particulier pour objet de nouvelles compositions pharmaceutiques caractérisées en ce qu'elles renferment un acide gras poly-insaturé ou un ester d'alcoyle de celui-ci, adsorbé sur une hydroxyalcoyl cellulose, en association ou en mélange avec un excipient ou un véhicule inerte, non toxique, pharmaceutiquement acceptable.

Elle a spécifiquement pour objet de nouvelles compositions pharmaceutiques caractérisées en ce qu'elles renferment un acide gras polyinsaturé ou un ester de celui-ci, adsorbé sur une hydroxypropylméthyl cellulose.

Les préparations obtenues selon le procédé de l'invention conviennent en particulier pour empêcher certains effets nocifs résultant de la suppression de la synthèse de prostaglandines, ou pour traiter ou prévenir notamment les effets ulcérogènes des agents anti-inflammatoires. Le niveau d'activité est du même ordre que celle des prostaglandines semi-synthétiques comme le Misoprostol mais, sans doute, en liaison avec la formation progressive ou limitée de PGE avec des effets secondaires bien plus atténués.

Ainsi, il est possible de rétablir lors de l'administration d'anti-inflammatoires non stéroïdiens, la synthèse de prostaglandines à partir de précurseurs naturels ou synthétiques, en utilisant une autre voie de métabolisme que celle des prostaglandine oxydases ou synthétases.

Les agents anti-inflammatoires administrés sont, soit des dérivés de l'acide salicylique tels que l'Aspirine, les amides de l'acide o.acétoxy benzoïque, soit des dérivés de l'acide anthranilique comme l'acide Niflumique ou ses esters, l'acide Tolfénamique, l'acide Méfénamique ou l'acide Flufénamique ou bien leurs esters, les acides indolyl acétiques comme l'Indométhacine, la sermétacine ou l'acémétacine; les acides phényl propioniques comme le Ketoprofène, le Pirprofène ou le Diclofénac ou bien encore les pyrazolones, comme la phénylbutazone ou l'oxyphénylbutazone, et les oxicams.

C'est ainsi que l'administration de la combinaison selon l'invention, associée à de l'aspirine, du diclofénac ou du piroxicam, diminue notablement la survenue d'effets secondaires chez les patients qui restent sous traitement par un anti-inflammatoire non stéroïdien.

Pour la voie topique, les compositions selon l'invention trouvent un emploi sous forme de crèmes, de lotions, de laits, de poudres aromatisées ou parfumées ou, d'une manière générale, toute présentation cosmétologique ou dermatologique usuelle.

Les compositions, selon l'invention, constituent encore un mode d'administration des précurseurs de prostaglandines, notamment E1, ces prostaglandines peuvent, dès lors, exprimer toutes leurs activités notamment dans les différents métabolismes.

Les exemples suivants illustrent l'invention sans toutefois la limiter :

### EXEMPLE I

| **Dispersion à 1% d'acide dihomo γ-linolénique** | |
|---|---|
| Acide dihomo γ-linolénique | 1 g |
| Hydroxypropylméthyl cellulose | 99 g |

L'acide dihomo γ-linolénique est dissout dans 10 ml d'acétone à 0° sous atmosphère d'azote. La solution ainsi réalisée est mélangée progressivement à l'hydroxypropylméthyl cellulose tout en maintenant le flux d'azote.

La pâte formée est recueillie et séchée sous vide à la température la plus basse possible.

La poudre est recueillie puis mélangée à du lactose et du talc pour former 1000 comprimés renfermant 1 mg d'acide dihomo γ -linolénique.

### EXEMPLE II

| **Dispersion à 2% d'acide dihomo γ-linolénique** | |
|---|---|
| Acide dihomo γ-linolénique | 2 g |
| Hydroxypropylméthyl cellulose | 100 g |
| Amidon de blé | 125 g |
| Lactose | 25 g |
| Stéarate de magnésium | 5 g |

pour 1000 comprimés achevés au poids moyen de 0,255 g et renfermant 2 mg d'acide dihomo γ-linolénique par comprimé.

L'acide dihomo γ-linolénique est dissout dans 25 ml d'acétonitrile fraîchement redistillé sur anti-oxydant et déshydraté par séchage sur sulfate de magnésium, préalablement dégazé par barbotage d'azote. La solution organique ainsi obtenue est filtrée sous azote, ajoutée progressivement à l'hydroxypropylméthyl cellulose, séchée sous vide puis tamisée pour obtenir une poudre homogène. Le mélange ainsi formé est ajouté à son tour à l'amidon de blé. On y ajoute, ensuite, le lactose et finalement le stéarate de magnésium. Le mélange final est granulé, broyé puis tamisé au tamis 200. La poudre fine ainsi recueillie est compressée pour former des comprimés d'un poids moyen de 0,255 g.

Les dispersions d'acide dihomo γ-linolénique, préparés selon les exemples I et II, se conservent pleinement. La perte après 2 ans de conservation à température ordinaire, est inférieure à 0,4%. On ne trouve pas trace de dérivé hydroxylé ou cétonique se trouvant normalement dans le produit après dégradation.

La posologie unitaire s'échelonne entre 0,2 mg et 1 mg d'acide dihomo γ-linolénique par prise d'essai. La posologie journalière chez l'homme s'échelonne entre 0,4 mg et 4 mg d'acide dihomo γ-linolénique divisé ou réparti dans l'hydroxypropylméthyl cellulose.

### EXEMPLE III

### Conservation d'un adsorbat d'acide γ-linolénique sur de l'hydroxypropylméthyl cellulose

### Introduction

L'objectif est d'obtenir une dispersion à 1% (m/m) d'acide γ-linolénique (abrégé en GLA) dans l'hydroxypropylméthyl cellulose (HPMC).

### Produits

L'acide gras polyinsaturé a été conservé au réfrigérateur. Un spectre RMN à 400 MHz du proton confirme que le produit n'a subit aucune altération durant son transport.

L'hydroxyalcoyl cellulose utilisée est le Méthocel E3Prem. (Dow Chemical) (HPMC).

La stabilité du mélange a d'abord été étudiée sous la forme initiale, c'est-à-dire non adsorbée, à température ambiante et sous agitation magnétique. On analyse le produit après dilution dans l'acétone (1 à 2mg dans 1ml d'acétone) et détermination par CPG. L'évaluation des chromatogrammes montre que :
- les deux premiers jours la stabilité de la préparation non adsorbée est bonne.
- le troisième jour, il commence à apparaître des produits de dégradation (96,6 % résiduel).
- le quatrième jour, la dégradation se poursuit (91 % résiduel).
- le septième jour, la dégradation est importante et, enfin, après 15 jours, il ne reste plus trace d'acide γ-linolénique initial. Dès le huitième jour, le produit devient opaque et jaunâtre. Sa viscosité augmente jusqu'à un état de pâte. L'acide γ-linolénique dilué dans l'acétone subit la même évolution.

### Mode opératoire de la préparation d'un adsorbat

On prélève, à l'aide d'une seringue, 0,05 g d'acide gras polyinsaturé que l'on place dans un ballon rodé à fond rond. Le liquide est dilué à l'aide de 2 g d'acétone pour analyse, puis 5 g d'HPMC sont dispersés manuellement dans la solution. Le tout est mélangé 10 mn au Rotavapor, sous azote. On établit le vide et l'évaporation dure environ 10 mn (absence d'odeur d'acétone). Le produit récupéré est légèrement broyé dans un mortier, afin d'éliminer les agglomérats. Finalement, la poudre (3,5 g) est mise dans un flacon à pénicilline et conservée à l'abri de la lumière, au réfrigérateur.

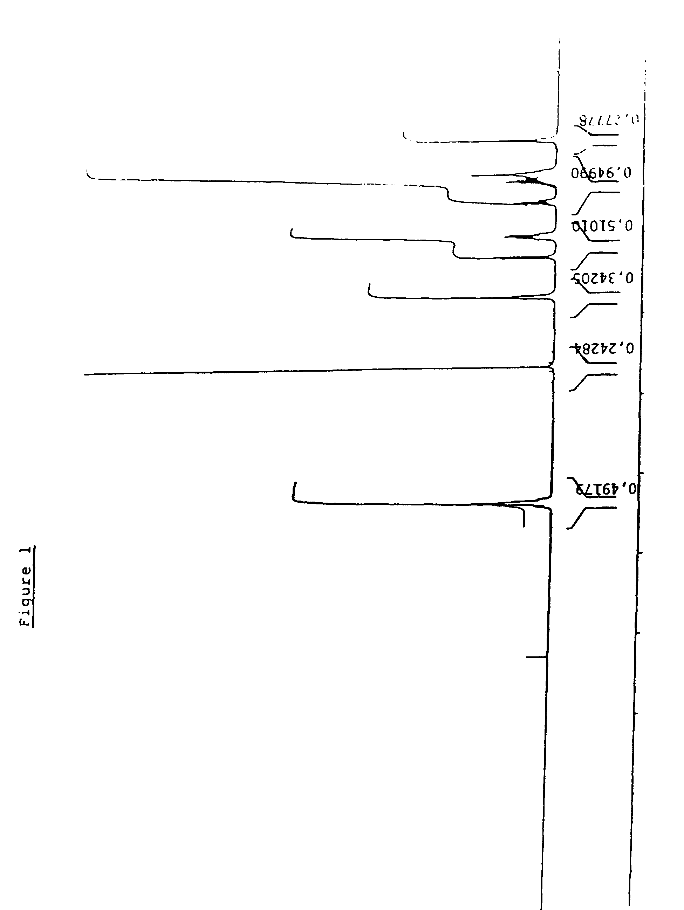

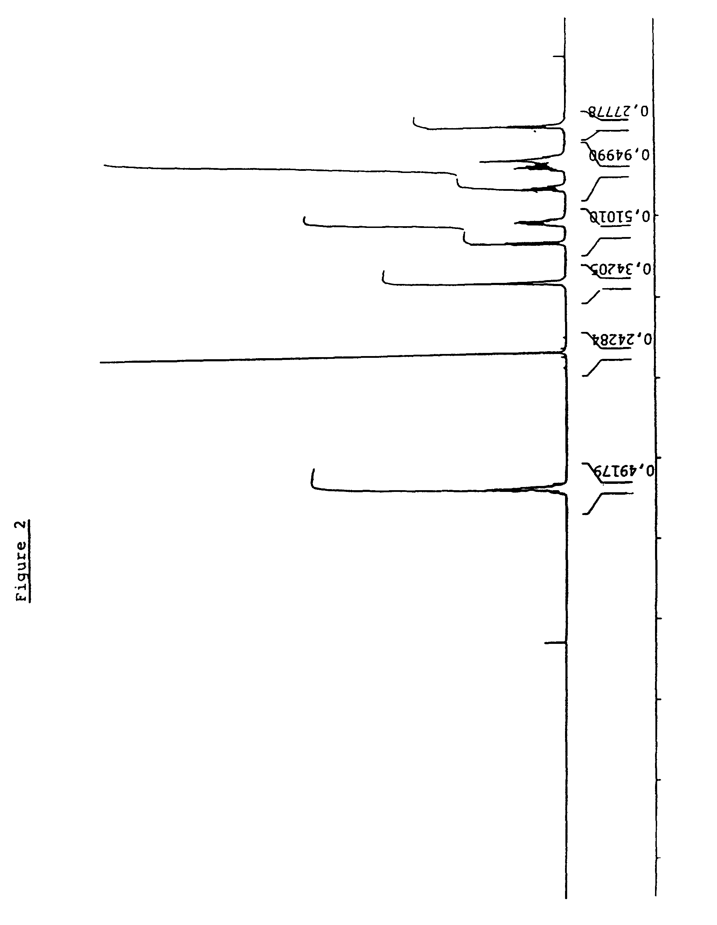

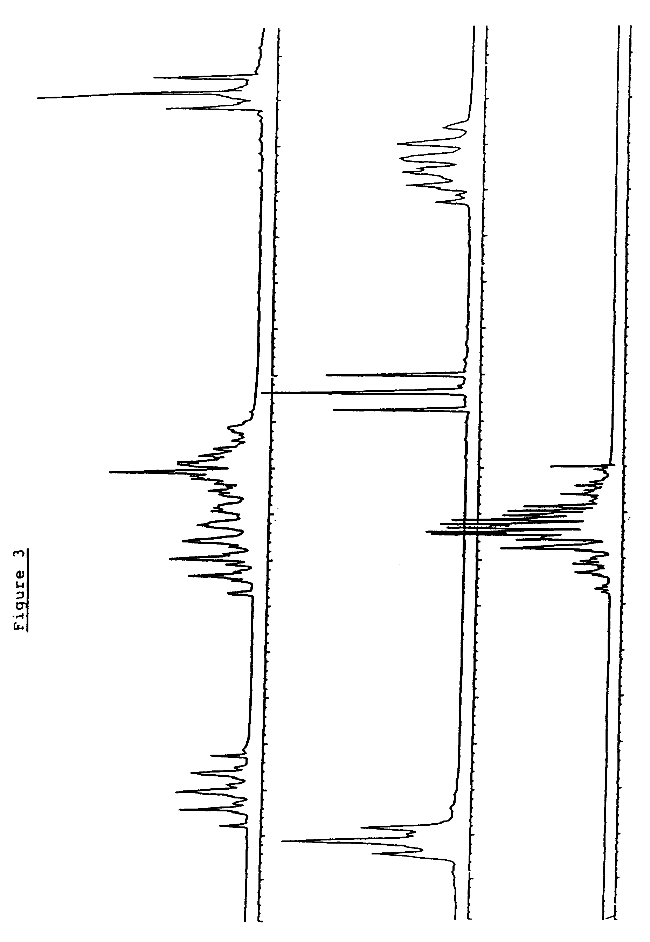

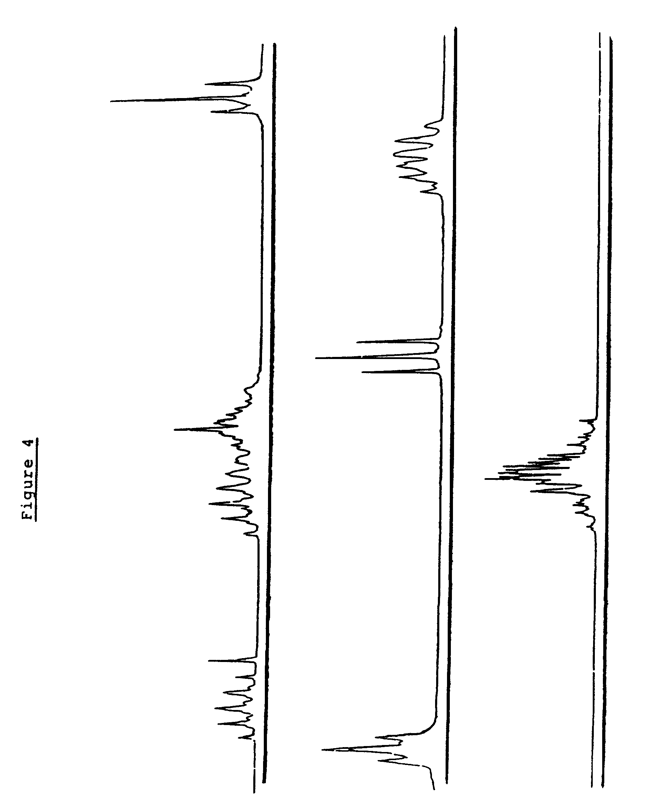

Les différents essais effectués fournissent des résultats tout à fait semblables (le lot récent est même plus pur), et confirment la structure du composé et sa stabilité. Les spectres RMN ci-annexés montrent que les produits n'ont subi aucune altération au cours du temps. La figure 1 est le spectre du γ-linolénate de méthyle adsorbé, déterminé au moment de la préparation et la figure 2 est le spectre RMN du même produit, après six mois de conservation à température ambiante. La figure 3 est un agrandissement du spectre de la figure 2.

### Vieillissement du produit initial

La stabilité a été étudiée sur le produit liquide tel quel à température ambiante et sous agitation magnétique. Pour l'analyse le produit est dilué dans l'acétone (1 à 2 mg dans 1 ml acétone), puis on fait une détermination du produit par CPG couplée à MS et par RMN ¹[H] ou ¹³[C].

Macroscopiquement l'apparence des poudres est totalement différente. Le produit non stabilisé, c'est-à-dire simplement dissout dans l'acétone à température ambiante prend l'aspect d'une pâte de couleur ocre.

### RESULTATS et COMMENTAIRES

Les spectres RMN, ci-joints, établis en 1995 (avec leurs agrandissements), montrent que les dérivés de l'acide γ-linolénique étudiés correspondent bien aux formules annoncées. En particulier, les signaux du spectre de l'extrait de γ-linolénate de méthyle sont les même que ceux du produit original, dont le spectre avait été obtenu en 1991 à l'époque de la préparation de la dispersion.

Dans tous les cas, les doubles liaisons sont bien présentes dans la zone 2-3 ppm et aucun signal (vers 5-6 ppm) n'indique d'oxydation des produits. Par ailleurs, la comparaison du spectre résultant de l'extraction du véhicule avec ceux des extraits des dispersions montre que le signal à 1,2 ppm et ceux situés entre 3 et 4,5 ppm sont attribuables à l'hydroxypropylméthylcellulose.

### CONCLUSION

L'examen des spectres RMN correspondant aux extraits des trois dispersions indique qu'aucun des dérivés n'a subi de dégradation au cours de la conservation.

### EXEMPLE IV

| **Exemples de formulations cosmétologiques** | | | | |
|---|---|---|---|---|
| | CREME JOUR NEUTRE | CREME JOUR à 1% d'ester méthylique d'acide γ-linolénique | CREME JOUR à 10% d'ester méthylique d'acide γ-linolénique | CREME JOUR à 1% d'acide dihomo γ-linolénique |
| ASPECT | lisse, brillant | idem | idem | idem |
| COULEUR | blanche | idem | ivoire | idem |
| ODEUR | caractéristique | idem | grasse | idem |
| TOUCHER | doux | très doux | trop collant | très doux |
| pH à 20°C | 7,1 | 7,1 | 7,1 | 7,1 |
| VISCO.RVT sp 4,20 t/mn | 25000 cps | 28000 cps | 40000 cps | 27500 cps |
| STABILITE 42°C | stable 15j | parfaitement stable | parfaite | parfaite |
| 50°C | stable 5j | parfaitement stable | parfaite | parfaite |

| | LAIT CORPOREL NEUTRE | LAIT CORPOREL à 1% d'ester méthylique d'acide γ-linolénique | LAIT CORPOREL à 10% d'ester méthylique d'acide γ-linolénique | LAIT CORPOREL à 1% d'acide dihomo γ-linolénique |
|---|---|---|---|---|
| ASPECT | lisse, brillant | idem | idem | idem |
| COULEUR | blanche | idem | ivoire | idem |
| ODEUR | caractéristique | idem | grasse | idem |
| TOUCHER | doux | plus onctueux | trop collant | plus onctueux |
| pH à 20°C | 6,5 | 6,5 | 6,5 | 6,5 |
| VISCO.RVT sp 4,20 t/mn | 10000 cps | 12000 cps | 20000 cps | 12000 cps |
| STABILITE 42°C | stable 15j | parfaite | parfaite | parfaite |
| 50°C | stable 5j | parfaite | parfaite | parfaite |

Les essais de formulation cosmétique ont montré qu'alors que des laits corporels ou des crèmes de jour n'étaient stables à 42 ou à 50°C, que pendant une durée limitée, les préparations, selon l'invention, dans les mêmes conditions, se conservent parfaitement.

En particulier, même si les préparations renfermant 10 % de γ-linolénate de méthyle ne présentent pas des propriétés optimales quant à la texture ou à la consistance, il apparaît clairement que leur degré de stabilité demeure inchangé.

De même il est possible d'utiliser des adsorbats plus concentrés en γ-linolénate de méthyle et ainsi d'introduire moins d'hydroxypropylmethyl cellulose dans la préparation. L'hydroxypropylmethyl cellulose devient génante si elle est supérieure à 10 % dans la formulation cosmétique et, en particulier, elle augmente trop la viscosité de la préparation.

De la même façon, on peut introduire dans les compositions cosmétiques des adsorbats concentrés à 10 % en acide gras poly-insaturé et réaliser après dilution des concentrations plus faibles de l'ordre de 1 % en hydroxypropylmethyl cellulose.

## Revendications

1. Procédé de stabilisation d'acides gras polyinsaturés ou de leurs esters, par inclusion dans un matériau à base d'hydrate de carbone en présence d'un solvant,
caractérisé en ce qu'on disperse un acide gras non saturé dans un solvant organique, de préférence non hydroxylé, et que l'on ajoute à cette dispersion une hydroxyalkylcellulose, éventuellement en solution dans un solvant inerte, puis on évapore à sec, sous atmosphère de gaz protecteur, la solution ou la dispersion ainsi réalisée pour obtenir une préparation pulvérulente d'acide gras polyinsaturé.

2. Un procédé de stabilisation selon la revendication 1, dans lequel l'hydroxyalcoyl cellulose est une hydroxyéthyl ou une hydroxypropyl cellulose.

3. Un procédé de stabilisation selon la revendication 1, dans lequel l'hydroxyalcoyl cellulose est une (hydroxyalcoyl) alcoyl cellulose.

4. Un procédé de stabilisation selon la revendication 1 et la revendication 3, dans lequel l'hydroxyalcoyl cellulose est une (hydroxypropyl) méthyl cellulose.

5. Un procédé selon la revendication 1, dans lequel le solvant organique est un solvant non hydroxylé.

6. Un procédé selon la revendication 1, dans lequel le solvant organique, non hydroxylé, est l'acétonitrile ou l'acétone.

7. Nouvelles compositions pharmaceutiques à base d'acides gras polyinsaturés ou d'un de leurs esters d'alkyle ou de glycéryle adsorbé sur un hydrate de carbone caractérisées en ce qu'elles renferment comme principe actif une préparation pulvérulente stable obtenue selon la revendication 1, en association ou en mélange avec un excipient ou un véhicule inerte pharmaceutiquement acceptable.

8. Une composition pharmaceutique selon la revendication 7, dans laquelle le rapport pondéral acide gras poly-insaturé/hydroxyalcoyl cellulose varie entre 0,1 et 50%.

9. Une composition pharmaceutique selon la revendication 7 ou la revendication 8, dans laquelle le rapport pondéral acide gras polyinsaturé/hydroxyalcoyl cellulose varie entre 1 et 10%.

10. Une composition pharmaceutique selon la revendication 7, ou la revendication 8° dans laquelle la posologie unitaire s'échelonne entre 0,2 mg et 1 mg d'acide gras poly-insaturé.

11. Une composition pharmaceutique selon l'une des revendications 7 à 10, dans laquelle l'acide gras poly-insaturé est l'acide stéaridonique.

12. Une composition pharmaceutique selon l'une des revendications 7 à 10, dans laquelle l'acide gras poly-insaturé est l'acide α-linolénique.

13. Une composition pharmaceutique selon l'une des revendications 7 à 10, dans laquelle l'acide gras polyinsaturé est l'acide dihomo γ-linolénique.

14. Une composition pharmaceutique selon l'une des revendications 7 à 10, dans laquelle l'acide gras polyinsaturé est l'acide γ-linolénique.

15. Une composition pharmaceutique selon l'une des revendications 7 à 10, dans laquelle l'ester d'acide gras polyinsaturé est le triglycéride d'acide γ-linolénique.

16. Une composition pharmaceutique selon l'une des revendications 7 à 10, dans laquelle l'acide gras polyinsaturé est un analogue semi-synthétique ou synthétique de l'acide dihomo γ-linolénique (acide 8, 11, 14-eicosatétraénoique) substitué en position 2,3,4,5,18 ou 19.

17. Une composition pharmaceutique selon l'une des revendications 7 à 10 et 16, dans laquelle l'acide gras polyinsaturé est l'acide 19-méthyl 8,11,14-eicosatriénoique.

18. Procédé de réalisation de compositions pharmaceutiques selon l'une des revendications 7 à 17, dans lequel la préparation pulvérulente d'acide gras poly-insaturé ou d'un de ses esters d'alcoyle ou de glycéryle, obtenue selon le procédé de la revendication 1, est diluée ou mélangée à un excipient ou un véhicule inerte, non-toxique, pharmaceutiquement-acceptable.

19. Compositions cosmétiques pour les soins de la peau caractérisées en ce qu'elles renferment un acide gras polyinsaturé, un des ses esters d'alcoyle ou un de ses esters de glycéryle adsorbé sur une hydroxyalcoyl cellulose selon la revendication 1, associé ou mélangé à un ou plusieurs excipients ou véhicules inertes appropriés pour l'application sur la peau.

## Patentansprüche

1. Verfahren zur stabilisierung ungesattigte Fettsaüre oder deren Estern durch Einbringung in einem Material aus Kohlenstoffhydraten in Gegenwart eines Losungsmittels,
dadurch gekennzeichnet daß
man eine ungesättigten Fettsaüre in einem organischen Losungsmittel, vorzugsweise eine nicht-hydroxylierte, Lösungsmittel, verteilt
und man dieser Dispersion eine Hydroxyalkylcellulose zusetzt, gefalls in einer Lösung in einem inerten Losungsmittel, danach zu Trockenheit unter schutzgas Atmosphär die so hergestellte Losung oder die Dispersion verdampft um einem pulverformige Zubereitung von polyungesattigte Fettsaüre zu erzielen.

2. Verfahren zur Stabilisierung nach Anspruch 1, worin die Hydroxyalkylcellulose eine Hydroxyethyl - oder eine Hydroxypropyl Cellulose ist.

3. Verfahren zur Stabilisierung nach Anspruch 1, worin die Hydroxyalkylcellulose eine (Hydroxyalkyl) alkylcellulose ist.

4. Verfahren zur Stabilisierung nach Anspruch 1 und 3, worin die Hydroxyalkylcellulose eine (Hydroxypropyl) methylcellulose ist.

5. Verfahren nach Anspruch 1, worin das organische Lösungsmittel eine nicht-hydroxylierte Lösungsmittel ist.

6. Verfahren nach Anspruch 1, worin das nicht-hydroxylierte organische Lösungsmittel Acetonitril oder Aceton ist.

7. Neue pharmazeutische Zusammensetzungen aus polyungesättigten Fettsaüren oder einen ihren Alkyl- oder Glycerylestern auf einem Kohlenhydrate aufgenommen, worin sie als Wirkstoff eine pulverförmige stabilisierte nach Anspruch 1 hergestellte Zubereitung, in Abmischung oder in Kombination mit einem inerten pharmazeutisch - verträgbare Träger oder Vehikeln enthalten.

8. Pharmazeutische Zusammensetzung nach Anspruch 7, worin das Gewichtes verhältnis polyungesättigte Fettsäure - Hydroxyalkylcellulose zwischen 0,1 und 50 % schwankt.

9. Eine pharmazeutische Zusammensetzung nach Anspruch 7 oder 8, worin das Gewichtes verhältnis polyungesättigte Fettsäure / hydroxyalkylcellulose zwischen 1 und 10 % schwankt.

10. Eine pharmazeutische Zusammensetzung nach Anspruch 7 oder 8, worin die eigentliche Dosierung zwischen 0,2 mg und 1 mg polyungesättigte Fettsäure liegt.

11. Eine pharmazeutische Zusammensetzung nach einer der Ansprüche 7 bis 10, worin die polyungesättigte Fettsäure, Stearidonsäure ist.

12. Eine pharmazeutische Zusammensetzung nach einer der Ansprüche 7 bis 10, worin die polyungesättigte Fettsäure, α-Linolensäure ist.

13. Eine pharmazeutische Zusammensetzung nach einer der Ansprüche 7 bis 10, worin die polyungesättigte Fettsäure, Dihomo γ-Linolensäure ist.

14. Eine pharmazeutische Zusammensetzung nach einer der Ansprüche 7 bis 10, worin die polyungesättigte Fettsäure, γ-Linolensäure ist.

15. Eine pharmazeutische Zusammensetzung nach einer der Ansprüche 7 bis 10, worin der Ester einer polyungesättigte Fettsäure, der Triglyzerid γ-Linolensäure ist.

16. Eine pharmazeutische Zusammensetzung nach einer der Ansprüche 7 bis 10, worin die polyungesättigte Fettsäure eine semi-synthetische oder synthetische Analog der Dihomo γ-linolensäure die in Stellung 2, 3, 4, 5, 18 oder 19 substituiert ist.

17. Eine pharmazeutische Zusammensetzung nach einer der Ansprüche 7 bis 10 und Anspruch 16, worin die polyungesättigte Fettsäure, 19-Methyl 8, 11, 14-eicosatriensäure ist.

18. Verfahren zur Herstellung pharmazeutische Zusammensetzung nach einer der Ansprüche 7 bis 10, worin die pulverförmige Zubereitung von polyungesättigte Fettsäure oder einer seine Alkyl oder Glyzeryl estern nach dem Verfahren der Anspruch 1 hergestellt mit einem inerten, nicht toxischen pharmazeutisch - verträg - baren Träger oder Vehikeln verdünnt oder vermischt ist.

19. Kosmetische Zusammensetzungen für Hautpflege dadurch gekennzeichnet daß sie eine polyunsättigte Fettsäure, eine deren Alkylestern oder eine deren glyzerylestern auf einem Hydroxyalkylcellulose aufgenommen, nach Anspruch 1, in Abmischung oder Vermischung mit einem oder mehreren inerten Träger oder Vehikeln die für das Auftragen auf dem Hautgeeignet sind.

## Claims

1. Method of stabilizing polyunsaturated fatty acids or esters thereof, by including them in a material based of carbohydrate in the presence of a solvent,
wherein a non saturated fatty acid is dispersed in an organic solvent, preferably a non hydroxylated solvent, and to this dispersion an hydroxyalkylcellulose is added, eventually in solution in an inert solvent, then, under a protective gas atmospher, the thus achieved solution or dispersion is evaporated to dryness to obtain a pulverulent preparation of polyunsaturated fatty acid.

2. A method of stabilizing according to claim 1, wherein the hydroxyalkylcellulose is an hydroxyethyl- or hydroxypropyl cellulose.

3. A method of stabilizing according to claim 1, wherein hydroxyalkyl cellulose is an (hydroxyalkyl) alkyl cellulose.

4. A method of stabilizing according to claims 1 and 3, wherein the hydroxyalkyl cellulose is an (hydroxypropyl)methyl cellulose.

5. A method according to claim 1, where in the organic solvent is a non hydroxylated solvent.

6. A method according to claim 1, wherein the non hydroxylated organic solvent is acetonitrile or acetone.

7. New pharmaceutical compositions based of polyunsaturated fatty acids or one of their alkyl or glyceryl ester thereof, adsorbed on a carbohydrate, characterized in that they contain as active ingredient a stable pulverulent preparation obtained according to claim 1, combined to or admixed with, an excipient or a pharmaceutically acceptable inert vehicle.

8. A pharmaceutical composition according to claim 7, wherein the weight ratio of polyunsaturated fatty acids to hydroxyalkyl cellulose is varying between 0.1 and 50 %.

9. A pharmaceutical composition according to claim 7 or claim 8, wherein the weight ratio of polyunsaturated fatty acids to hydroxyalkyl cellulose is varying from 1 to 10 %.

10. A pharmaceutical composition according to claim 7 or claim 8 wherein unit posology ranges from 0.2 mg to 1 mg of polyunsaturated fatty acid.

11. A pharmaceutical composition according to claim 7 to 10, wherein polyunsaturated fatty acid is stearidonic acid.

12. A pharmaceutical composition according to one of claims 7 to 10, wherein the polyunsaturated fatty acid is α-linolenic acid.

13. A pharmaceutical composition according to one of claims 7 to 10, wherein the polyunsaturated fatty acid is dihomo γ-linolenic acid.

14. A pharmaceutical composition according to one of claims 7 to 10, wherein the polyunsaturated fatty acid is γ-linolenic acid.

15. A pharmaceutical composition according to one of claims 7 to 10, wherein the ester of polyunsaturated fatty acid is γ-linolenic acid triglycerid.

16. A pharmaceutical composition according to one of claims 7 to 10, wherein polyunsaturated fatty acid is a semi-synthetic or synthetic analog of dihomo γ-linolenic acid (8, 11, 14-eicosatetranoic acid), substituted in position 2,3,4,5,18 or 19.

17. A pharmaceutical composition according to one of claims 7 to 10 and 16, wherein the polyunsaturated fatty acid is 19-methyl 8,11,14-eicosatrienoic acid.

18. Method of production of pharmaceutical compositions according to one of claims 7 to 17, wherein the pulverulent preparation of polyunsaturated fatty acid or one of alkyl or glyceryl ester thereof, obtained according to the method of claim 1, is diluted or admixed with a non toxic, pharmaceutically acceptable diluent or an inert vehicle.

19. Cosmetic compositions for skin care wherein they contain polyunsaturated fatty acid, or one of their alkylester or glyceryl ester thereof adsorbed on an hydroxyalkyl cellulose according to claim 1, combined or admixed with one or several inert diluents or vehicles suitable for application on the skin.
